# EUROPEAN PATENT APPLICATION

(11) **EP 1 346 728 A1**
(43) Date of publication of application: **24.09.2003**
(21) Application number: 01982866.4
(22) Date of filing: 20.11.2001
(51) Int. Cl.: A61K 45/00, A61K 31/50, A61P 27/02, A61P 9/10, A61P 27/06, A61P 43/00, C07D 237/04

(54) **OPHTHALMOLOGICAL PREPARATIONS**

(30) Priority: 22.11.2000 JP 2000356798
(71) Applicant: Mitsubishi Pharma Corporation, Osaka-shi, Osaka 541-0046 (JP)
(72) Inventor: MANO, Tomiya, Ibaraki-shi, Osaka 567-0048 (JP); SOGO, Shunji, Yao-shi, Osaka 581-0016 (JP); INOUE, Eri, Himeji-shi, Hyogo 671-0021 (JP)
(74) Representative: ter Meer, Nicolaus
(86) International application number: JP0110129
(87) International publication number: WO02041917

(57) **Abstract**

The invention relates to an ophthalmic agent comprising, as an active ingredient, a compound having a cardiotonic action including a phenyl pyridazinone derivative or salt thereof, or a hydrate thereof or solvate thereof as a typical example. Specifically, the invention relates to a preventative and/or therapeutic agent for optic nerve diseases or retinal diseases.

## Description

### Technical Field

The present invention relates to an ophthalmic agent which comprises as an active ingredient a compound having a cardiotonic action including a phenyl pyridazinone derivative or a salt thereof, or a hydrate thereof or a solvate thereof as a typical example. Specifically, the invention relates to a preventative and/or therapeutic agent for an optic nerve disease or a retinal disease.

### Background Art

As for nerve cell death and neuroprotection in ophthalmologic field, it has been clarified by recent researched that nerve cell deaths occur through apoptosis in most optic nerve diseases and retinal diseases. Mechanism that induces optic nerve cell death in diseases of optic nerve and neural retina has not yet been fully clarified, but retinal ischemia is considered to be one of factors. Ischemia is defined as a lack or an insufficiency of tissue blood circulation, and hypoxemia resulting from reduced ocular blood flow due to increased intraocular pressure (IOP) is believed to play an important role in degeneration of optic nerve and neural retina. In view of the facts, therapeutic treatment of glaucoma at present is performed by lowering IOP by drug therapy or surgical therapy. Effectiveness of the therapies has been verified by extensive researches. However, in significant numbers of clinical cases, IOP cannot be sufficiently reduced, and in some cases, a reduction of IOP is ineffective. In the long view, there is a risk of blindness at a considerable rate even where a therapy is performed. Under the circumstances, establishment of a therapeutic strategy has been desired for treatment of glaucoma, in which a direct neuroprotection of optic nerve is focused from a viewpoint of circulation improvement and cell death suppression, as well as the effect of reduction of IOP (Exp. Eye Res. 69, 331-342 1999 and the like) Further, because circulation disorders in the retina caused by retinal artery occlusion or retinal vein occlusion lead to severe ocular function disorders and no suitable therapeutic means is available, an effective therapeutic agent for the diseases has been strongly desired.

Phenyl pyridazinone derivative is known as a cardiotonic agent (Japanese Patent Publications (Kokai) Nos. 60-126282 and 61-289032). As far as the present inventors are aware, no report has been made so far concerning an inhibitory action against nerve cell death in the neural retina and optic nerve or against retinal degeneration, as well as concerning an ophthalmic application thereof.

An object of the present invention is to provide a novel use of a compound having a cardiotonic action. More specifically, the object is to provide a novel preventative and/or therapeutic agent for optic nerve diseases and retinal diseases.

### Disclosure of the Invention

The present inventors conducted thorough researches on the mechanism of nerve cells death in the optic nerve and neural retina. They considered that if a method of treatment is developed achieves suppression of cell death, the method can improve treatment effectiveness for these intractable diseases, and conducted various researches to achieve the foregoing object. As a result, the inventors found that a compound, having been used so far only as a cardiotonic agent, has an inhibitory action on ganglion cell death, as well as an inhibitory action on degeneration of the retina, and in the consequence, the compound can be used as a ophthalmic agent. The present invention was achieved on the basis of these findings.

The gist of the present invention thus relates to an ophthalmic agent which comprises a compound having a cardiotonic action as an active ingredient.

Preferred embodiments of the present invention include the agent wherein the compound has enhancing action on calcium sensibility; the agent wherein the compound is a phenyl pyridazinone derivative represented by the following general formula (1) or (2) or a salt thereof, or a hydrate thereof or a solvate thereof: 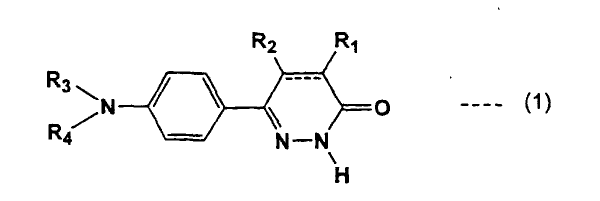 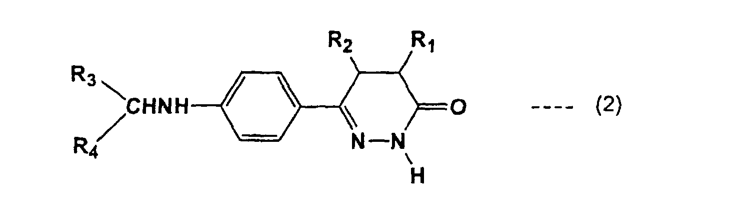 (wherein R₁ and R₂ independently represent hydrogen atom, halogen atom, an alkyl group, amino group, carboxyl group, nitro group, cyano group, trifluoromethyl group, an acylamino group, or an alkoxyl group; R₃ represents hydrogen atom, an acyl group, or an alkyl group; R₄ represents a 5 or 6-membered heterocyclic ring which may be substituted on the ring); the agent wherein the compound is a phenyl pyridazinone derivative represented by the following general formula (3) or a salt thereof, or a hydrate thereof or a solvate thereof: 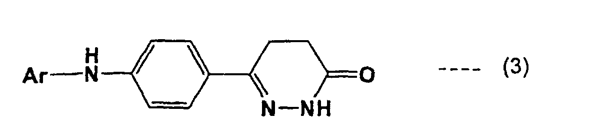 (wherein A is a 5 or 6-membered heterocyclic ring containing 1 to 3 nitrogen atoms, wherein said ring may optionally be substituted with at least one substituent selected from the group consisting of an alkyl group, cyano group, hydroxyl group, an alkoxyl group, amino group, an alkylamino group, an dialkylamino group, an acylamino group, carboxyl group, an alkoxycarbonyl group, and carbamoyl group); the agent wherein the compound is represented by the general formula (1) or (2) wherein R₁, R₂ and R₃ independently represent hydrogen atom, methyl group, or ethyl group, and R₄ represents 2-pyridyl group, 4-pyridyl group, 2-pyrimidyl group, or 4-quinolyl group; and the agent wherein the compound is represented by the general formula (1), wherein R₁, R₂ and R₃ are hydrogen atoms, and R₄ is 4-pyridyl group.

The second gist of the present invention relates to a pharmaceutical composition for optic nerve diseases or retinal diseases, which comprises a compound having a cardiotonic action as an active ingredient. Preferred embodiments thereof include the composition wherein the compound has enhancing action on calcium sensibility; the composition wherein the optic nerve disease is selected from ischemic optic neuropathy or glaucoma; and the composition wherein the retinal disease is selected from retinal vein occlusion, retinal artery occlusion, and diabetic retinopathy.

The third gist of the present invention relates to a preventative and/or therapeutic agent for optic nerve diseases or retinal diseases which comprises a compound having a cardiotonic action as an active ingredient. Preferred embodiments thereof include the agent wherein the compound has enhancing action on calcium sensibility; the agent wherein the optic nerve disease is selected from ischemic optic neuropathy or glaucoma; and the agent wherein the retinal disease is selected from retinal vein occlusion, retinal artery occlusion, and diabetic retinopathy; and the agent is selected from orally available agents, injections, eye drops, ointments, sustained release agents for placing inside of eyelid, or ophthalmic circulatory solutions.

Further gists of the present invention include an inhibitor against retinal ganglion cell death which comprises a compound having a cardiotonic action as an active ingredient; an agent for protection of retinal ganglion cell which comprises a compound having a cardiotonic action as an active ingredient; a method for inhibiting retinal ganglion cells death by using a compound having a cardiotonic action; a method of protecting retinal ganglion cells by using a compound having a cardiotonic action; a retinal degeneration inhibitor which comprises a compound having a cardiotonic action as an active ingredient; and a method for inhibiting retinal degeneration by using a compound having a cardiotonic action. Among them, preferable embodiments include those wherein the compound has enhancing action on calcium sensibility, and those wherein the retinal degeneration is caused by retinal ischemia.

### Brief Explanation of the Drawings

Figure 1 shows the inhibitory effect on retinal ganglion cell death caused by glutamate in Example 1.
Figure 2 shows the inhibitory effect on retinal degeneration induced by transient retinal ischemia in Example 2.

### Best Mode for Carrying Out the Invention

The present invention will be explained in detail below.

In the present invention, a compound having a cardiotonic action is not particularly limited as long as the compound has an action of increasing developed tension in isolated cardiac muscle samples of various animals as shown in the Reference Examples below. Whether or not a compound has an action of increasing developed tension in isolated cardiac muscle samples deriving from various animals is examined by methods shown in the Reference Examples, and where the compound has an action of increasing the developed tension, the compound can be judged as a compound having a cardiotonic action according to the definition of the present invention.

Examples of such compounds include compounds as represented by the above general formula (1) or (2). Examples of the compound include those wherein, in the general formula (1) or (2), R₁ and R₂ independently represent hydrogen atom, a halogen atom, an alkyl group, amino group, carboxyl group, nitro group, cyano group, trifluoromethyl group, an acylamino group, or an alkoxyl group; R₃ represents hydrogen, an acyl group, or an alkyl group; R₄ represents a 5 or 6-membered heterocyclic ring which may optionally be substituted on the ring. Among them, preferred compounds include those represented by the general formula (3), and those represented by the general formula (1) or (2) wherein Ri, R₂, and R₃ independently represent hydrogen atom, methyl group, or ethyl group, and R₄ represents 2-pyridyl group, 4-pyridyl group, 2-pyrimidyl group, or 4-quinolyl group. A particularly preferred compound according to the present invention includes a compound represent by the general formula (3) wherein A is 4-pyridyl group, i.e. 6-[4-(4-pyridylamino) phenyl]-4,5-dihydro-3 (2H)pyridazinone (hereinafter, also referred to as "MCI-154") or a salt thereof, or a hydrate thereof or a solvate thereof.

As for the mechanism of cardiotonic action of MCI-154, it is reported that this action is based on an enhancing action on calcium sensibility in contraction filaments in the cardiac muscle, as described in Japanese Journal of Pharmacology, 50, 411-419 (1989). Therefore, according to the present invention, a compound having the enhancing action on calcium sensibility can be used as an active ingredient of the ophthalmic agent of the present invention. In the above publication, each effect of MCI-154, sulmazole, adibendan, and pimobendan on a tension developed by addition of calcium (pCa 5.8) is described by using a skinned fiber specimen prepared by immersion of left ventricle papillary muscle of a guinea pig in saponin (250 µg/ml) for 30 minutes. Where a compound is examined in respect of the enhancing action on calcium sensibility by such a method and the compound is proved to have the calcium sensitizing action, the compound can be judged to have the enhancing action on calcium sensibility defined according to the present invention.

These compounds are described in the Japanese Patent Publications (Kokai) Nos. 58-8015, 58-8016, 58-140076, and 60-126282, as well as in Journal of Medicinal Chemistry, 1987, vol. 30, No. 7, 1157-1161, and they can be manufactured by the methods described in these publications and the like or similar methods thereto.

Salts of these compounds are not particularly limited as long as they are pharmaceutically acceptable salts. Examples include salts with mineral acids such as hydrochloric acid, phosphoric acid and the like, and salts with organic acids such as lactic acid, acetic acid and the like.

Further, in the present invention, the compounds mentioned above can be used as a hydrate or a solvate. A solvent of the solvate is not particular limited so long as it is pharmaceutically acceptable.

The present invention will be explained below as for an example using MCI-154. However, is should be noted that when other compounds are used as active ingredients, they can be appropriately used by referring to the explanations below.

When the active ingredient of the medicament of the present invention is clinically applied, MCI-154 or a pharmaceutically acceptable salt thereof, per se, can be used, or the substance can be used as a pharmaceutical composition by formulation with a pharmaceutically acceptable carrier. Where the medicament is used as an eye-drop, it is preferable to administer 1 to 2 drops of the above compound at 0.1 to 100 mg/ml, once to several times a day. Where the medicament is used orally, it is preferable to administer 0.01 to 100mg/kg of the above compound 1 to 3 times a day. For an intravenously injection, it is preferable to administer 0.01 to 200 mg of the compound 2 to 5 times a day, or the above dose may be administered continuously by drip infusion. Further, for rectal administration, it is preferable to administer 0.01 to 100mg/kg of the compound 1 to 3 times a day. Further, the compound can also be used by adding to an ophthalmic circulatory solution in the eye. The above administration doses can be appropriately increase or decreased depending on age, clinical conditions, sexuality, and symptoms.

Eye-drops include aqueous eye-drops, non-aqueous eye-drops, suspension-type eye-drops, emulsion-type eye-drops, eye ointments and the like. Manufactures of the eye-drops can be performed by using aqueous media such as sterilized water or physiological saline, or non-aqueous media such as vegetable oil including cotton seed oil, soy bean oil, sesame seed oil, peanut oil and the like, and dissolving or suspending MCI-154 or a pharmaceutically acceptable salt thereof in the medium. For the preparation, pharmaceutically acceptable isotonic agents, pH modifiers, viscosity controlling agents, suspending agent, emulsifying agents, preservation agents or the like may be suitable added as required.

For manufactures of other formulations, MCI-154 or a pharmaceutically acceptable salt thereof can be used as a composition comprising an ordinarily used carrier for formulation, i.e., an excipient or other additive. The carrier may be solid or liquid.

Where a solid carrier is used, the medicament may be in a form of a tablet, a powder, a granule, a hard gelatin capsule, a suppository, a troche or the like. The solid carrier may be used in any amount. Preferably, the amount may be chosen so as to be from about 1 mg to about 1 g.

Where a liquid carrier is used, the medicament may be prepared in a form of a syrup, an emulsion, or a soft gelatin capsule, or a sterilized injection solution filled in an ampoule or an aqueous or non-aqueous suspension.

For formulation according to the present invention, it is also preferred that MCI-154 or a pharmaceutically acceptable salt thereof is formulated as a sustained-release agent by preparation of a cyclodextrin clathrate or encapsulation in a liposome.

The ophthalmic agent of the present invention thus obtained inhibits/protects against retinal ganglion cell death and suppresses retinal degeneration, in particular, degeneration caused by retinal ischemia, as is shown in the following Examples. Accordingly, the agent can be used as a medicament for preventive or therapeutic treatment of optic nerve diseases or retinal diseases. According to the present invention, the optic nerve diseases are not particularly limited as long as they are caused by retinal ganglion cell death, and examples include ischemic neuritis optica and glaucoma. According to the present invention, retinal diseases are not particularly limited as long as they are caused by retinal degeneration, and examples include retinal vein occlusion, retinal artery occlusion, diabetic retinopathy and the like.

### Examples

The present invention will be explained more specifically by way of Examples. However, the present invention is not limited to the examples so far that it exceeds the gist thereof. MCI-154 used in the following examples was manufactured according to the method described in Example 1 of Japanese Patent Publication (Kokai) No. 60-126282.

### Example 1: Inhibitory effect against retinal ganglion cell death caused by glutamate

### Experimental Method

In accordance with the method of Otori et al. (Investigative Ophthalmology & Visual Science, Vol. 39, No. 6, 1998), retinal ganglion cells were isolated from a 6 day-old Wister rat, and approximately 300 cells were plated on each cover glass having a diameter of 12 mm with poly-L-lysine and laminin coatings, that was inserted in each well of a 24-well culture plate, and then were cultured for 24 hours. The cells were divided into an untreated group, a control group, and medicament-treated group, each consisting of 2 wells. To the untreated and control groups, a basic culture medium was added, and to the medicament-treated groups, MCI-154 was added at various concentrations. After 4 hours of culture, 250 µM of glutamate was added to all of the groups except for the untreated group, followed by further culture for 48 hours. After the culture for 48 hours, cells were stained with 1 µM calcein acetoxymethyl ester, and the number of cells with neurites growing more than twice as long as the cell body diameter was counted as viable cells under a fluorescence microscope. Independent three experiments were performed in duplicate.
Medicament-treated group: 10⁻⁷ M, 10⁻⁶ M, and 10⁻⁵ M MCI-154
Basic culture medium: Neurobasal (Gibco, Grand Island, NY) including 1 mM glutamine, 10 µg/ml gentamycin, and 40 ng/ml BDGF, CNTF, and b-FGF.

### Experimental Results

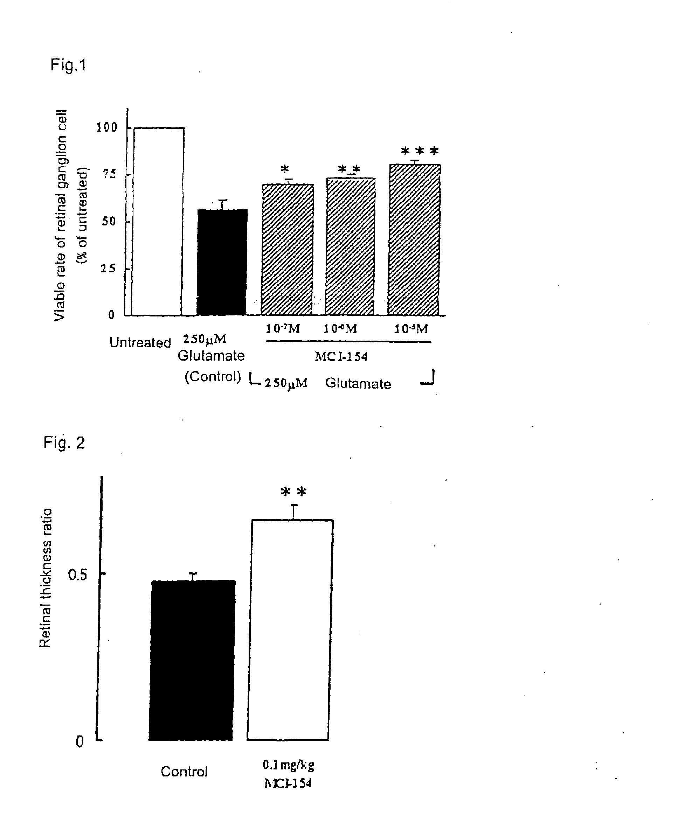

Results are shown in Fig.1. Figure 1 shows percentages of viable cells in each group relative to the number of viable cells in the untreated group. Date represent the means S.E.M. of three independent experiments, which were performed in duplicate. In Fig.1, *, ** and *** represent significant differences of(p<0.05), (p<0.01), and (p<0.001), respectively, relative to the control group.

From these results, it is clearly demonstrated that MCI-154 significantly inhibits retinal ganglion cell death induced by glutamate.

### Example 2: Inhibitory effect against retinal degeneration induced by transient retinal ischemia

### Experimental Method

Wister male rats (body weight of about 200 g) was used. Each animal was subjected to sufficient warming treatment by using a thermal plate under anesthesia, and the head was fixed by using a stereotaxic apparatus. In order to induce transient retinal ischemia, IOP was maintained at 130 mmHg for 50 minutes by injecting intraocular irrigating solution into the anterior chamber via the cornea using an infusion tube and 30 G injection needle which were connected to a bottle held at 174 cm above the eyes (ocular hypertension treatment) according to the method of Akaike (Folia Pharmacologica Japonica, 111, pp.97-104(1998)). The ocular hypertension treatment was performed only on the right eye, and the left eye was left untreated. Just before and immediately after the ocular hypertension treatment, a physiological saline or a dose of 0.1 mg/kg of MCI0154 was injected to the caudal vein. Seven days after the ocular hypertension treatment, the animal was sacrificed under anesthesia, and eyeballs were isolated and a light microscopic specimens of horizontal sections of retina were prepared. Under a microscope, thickness of the retina (from inner plexiform layer to outer plexiform layer) was measured using a micrometer.

### Experimental Results

Fig. 2 shows the retinal thickness ratios calculated as [Retinal thickness in the ocular hypertension treated eye/Retinal thickness in the normal eye, retinal thickness: total thickness of from inner plexiform layer to inner plexiform layer] 7 days after the ocular hypertension treatment. Data represent means ± SEM (standard errors), and the number of rats for each group was 10. In Fig. 2, ^{**} indicates a significant difference relative to the control group (p<0.01).

From these results, it was clearly demonstrated that MCI-154 significantly inhibits retinal degeneration caused by transient retinal ischemia.

### Reference Examples

### Method using an isolated and cross-circulated papillary muscle preparation of dog

An isolated and cross-circulated papillary muscle preparation of dog was prepared in accordance with the method of Endo and Hashimoto (American J. Physiol, 218, 1459-1463, 1970). The effect of the compound was measured by closely intraarterially injecting the compound dissolved in a solvent to the papillary muscle for the purpose of recording its effect on contraction of papillary muscle. Rate of increase in contraction of papillary muscle contractive is shown in the following Table.

**Table 1**

| Compound | Contraction of papillary muscle of dog | |
|---|---|---|
| | Dose (µg i.a.) | Increase (%) |
| MCI-154 | 10 | 13.2 |
| | 30 | 15.4 |

### 2. Method using an isolated left atrium of guinea pig.

A left atrium was isolated from a male guinea pig with 200-300 g of body weight soon after striking on the back of its head. The mitral orifice was fixed to the bottom of an organ bath filled with 30 ml of Krebs-Henseleit solution maintained at 35°C. A gas mixture comprising 95% O₂ and 5% CO₂ was passed through the Krebs-Henseleit solution in the organ bath. Isometric tension was measured by connecting a left auricle of heart and a transducer by a yarn. A resting tension of 0.5 g was given to the atrium, which was then electrically driven with square pulses with duration of 1 msec and voltage of 1.5 times as much as threshold in the rate 2 Hz via dipolar platinum electrodes. After stabilizing the atrium for 30 minutes from its preparation, the compound dissolved in a solvent was added to the organ bath to measure its effect. The rate of increase in contraction of left atrium is shown in the following Table 2.

**Table 2**

| Compound | Contraction of left atrium of guinea pig | |
|---|---|---|
| | Dose (g/ml) | Increase (%) |
| MCI-154 | 10⁻⁵ | 115 |
| | 3 x 10⁻⁵ | 138 |

### Method using an anaesthetized dog

Male and female mongrel dogs with body weight of 8-15 kg were used. A dog was anaesthetized with intravenous injection of 30 mg/kg of sodium pentobarbital and practiced artificial respiration. The dog was thoractomized between forth and fifth costa which was cut off. The pericardium was incised to expose heart. Blood flow through the aorta, which was measured with an electromagnetic blood flowmeter whose probe was attached to the ascending aorta, was used as an approximate index of cardiac output (CO). Left ventricular pressure (LVP) was measured with a Miller Catheter-tip pressure transducer and the first derivative of the LVP (dP/dt) was measured with a differentiater. Contraction of right ventricular muscle (Cont) was determined with a strain-gauge attached to the wall. Systemic blood pressure was measured from the left femoral artery. Heart rate was measured with electrocardiogram (lead II) and a cardiotachometer. The compound dissolved in a solvent was administered intravenously from a left femoral vein. Maximum value of dP/dt (dPdt max) and rate of increase in Cont and CO are shown in the following Table 3.

**Table 3**

| Compound | Anaesthetized dog | | | |
|---|---|---|---|---|
| | Dose (µg/kg i.v.) | Increase of DP/dt max (%) | Increase of Cont (%) | Increase of CO (%) |
| MCI-154 | 10 | 45 | 23 | 13 |
| | 30 | 93 | 57 | 28 |

### Industrial Applicability

According to the present invention, a novel use of a compound having a cardiotonic action is provided. More specifically, novel preventive and/or therapeutic agent for optic nerve diseases and retinal diseases can be provided.

The present application was filed claiming priority on Japanese Patent Application No. 2000-356798.

## Claims

1. An ophthalmic agent which comprises a compound having a cardiotonic action as an active ingredient.

2. The ophthalmic agent according to claim 1, wherein the compound has enhancing action on calcium sensibility.

3. The ophthalmic agent according to claim 1 or 2, wherein the compound is a phenyl pyridazinone derivative represented by the following general formula (1) or (2) or a salt thereof, or a hydrate thereof or a solvate thereof: 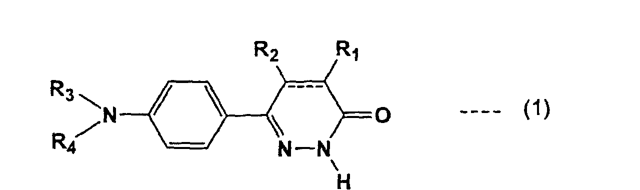 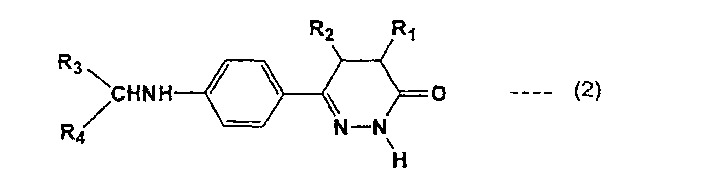 wherein R₁ and R₂ independently represent hydrogen atom, a halogen atom, an alkyl group, amino group, carboxyl group, nitro group, cyano group, trifluoromethyl group, an acylamino group, or an alkoxyl group; R₃ represents hydrogen atom, an acyl group, or an alkyl group; and R₄ represents a 5 or 6-membered heterocyclic ring which may be substituted on the ring.

4. The ophthalmic agent according to any one of claims 1 to 3, wherein the compound is a phenyl pyridazinone derivative represented by the following general formula (3) or a salt thereof, or a hydrate thereof or a solvate thereof: 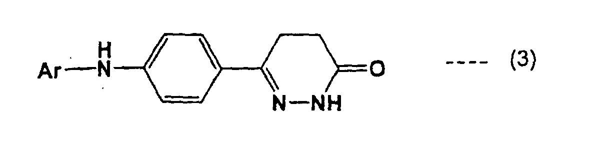 wherein A is a 5 or 6-membered heterocyclic ring containing 1 to 3 nitrogen atoms, wherein said ring may optionally be substituted with at least one substituent selected from the group consisting of an alkyl group, cyano group, hydroxyl group, an alkoxyl group, amino group, an alkylamino group, an dialkylamino group, an acylamino group, carboxyl group, an alkoxycarbonyl group, and carbamoyl group.

5. The ophthalmic agent according to any one of claims 1 to 3, wherein the compound is represented by the general formula (1) or (2) according to claim 3 wherein R₁, R₂ and R₃ independently represent hydrogen atom, methyl group, or ethyl group, and R₄ represents 2-pyridyl group, 4-pyridyl group, 2-pyrimidyl group, or 4-quinolyl group.

6. The ophthalmic agent according to any one of claims 1 to 5, wherein the compound is represented by the general formula (3) according to claim 4, wherein A is 4-pyridyl group.

7. A pharmaceutical composition for an optic nerve disease or a retinal disease, which comprises a compound having a cardiotonic action as an active ingredient.

8. The pharmaceutical composition according to claim 7, wherein the compound has enhancing action on calcium sensibility.

9. The pharmaceutical composition according to claim 7 or claim 8, wherein the optic nerve disease is selected from ischemic optic neuropathy and glaucoma.

10. The pharmaceutical composition according to clam 7 or 8, wherein the retinal disease is selected from retinal vein occlusion, retinal artery occlusion, and diabetic retinopathy.

11. A preventative and/or therapeutic agent for an optic nerve disease or a retinal disease which comprises a compound having a cardiotonic action as an active ingredient.

12. The preventative and/or therapeutic agent according to claim 11, wherein the compound has enhancing action on calcium sensibility.

13. The preventative and/or therapeutic agent according to claim 11 or claim 12, wherein the optic nerve disease is selected from ischemic optic neuropathy and glaucoma.

14. The preventative and/or therapeutic agent according to claim 11 or claim 12, wherein the retinal disease is selected from retinal vein occlusion, retinal artery occlusion, and diabetic retinopathy.

15. The preventative and/or therapeutic agent according to any one of claims 11 to 14, which is selected from an orally available agent, an injection, an eye drop, an ointment, a sustained release agent for placing inside of eyelid, and an ophthalmic circulatory solution.

16. An inhibitor against retinal ganglion cell death which comprises a compound having a cardiotonic action as an active ingredient.

17. The inhibitor against retinal ganglion cell death according to claim 16, wherein the compound has enhancing action on calcium sensibility.

18. An agent for protection of retinal ganglion cell which comprises a compound having a cardiotonic action as an active ingredient.

19. The agent for protection of retinal ganglion cell according to claim 18, wherein the compound has enhancing action on calcium sensibility.

20. A method for inhibiting retinal ganglion cells death by using a compound having a cardiotonic action.

21. The method for inhibiting retinal ganglion cells death according to claim 20, wherein the compound has enhancing action on calcium sensibility.

22. A method for protecting retinal ganglion cells by using a compound having a cardiotonic action.

23. The method for protecting retinal ganglion cells according to claim 22, wherein he compound has enhancing action on calcium sensibility.

24. An agent for suppression of retinal degeneration which comprises a compound having a cardiotonic action as an active ingredient.

25. The agent for suppression of retinal degeneration according to claim 24, wherein the retinal degeneration is caused by retinal ischemia.

26. The agent for suppression of retinal degeneration according to claim 24 or claim 25, wherein the compound has enhancing action on calcium sensibility.

27. A method for inhibiting retinal degeneration by using a compound having a cardiotonic action.

28. The method for inhibiting retinal degeneration according to claim 27, wherein the retinal degeneration is caused by retinal ischemia.

29. The method for inhibiting retinal degeneration according to claim 27 or claim 28, wherein the compound has enhancing action on calcium sensibility.
